# NEW EUROPEAN PATENT SPECIFICATION

(11) **EP 2 427 168 B2**
(45) Date of publication and mention of the opposition decision: **29.06.2022**
(45) Mention of the grant of the patent: 31.12.2014
(21) Application number: 10713340.7
(22) Date of filing: 12.04.2010
(51) Int. Cl.: A61K 8/27, A61K 8/49, A61K 8/73, A61Q 5/02, A61Q 5/00

(54) **Hair Care Composition comprising zinc-based anti-dandruff agent, conazole fungicide and cationic deposition polymer**
Haarpflegezusammensetzung enthaltend ein Zink-basiertes Antischuppenmittel, ein Conazole-Fungizid und ein kationisches Polymer
Composition de soin capillaire contenant un agent antipelliculaire à base de zinc, un fongicide de type conazole et un polymère cationique

(30) Priority: 08.05.2009 EP 09159807
(43) Date of publication of application: 14.03.2012
(73) Proprietor: Unilever IP Holdings B.V., 3013 AL Rotterdam (NL); Unilever Global IP Limited, Wirral, Merseyside CH62 4ZD (GB)
(72) Inventor: CHANG, Wanlin, Shanghai 200335 (CN); JAYASWAL, Amit, Shanghai 200335 (CN); LI, Zheng Rong, Shanghai 200335 (CN); SAMRAN, Busaraporn, 74000 Thailand (TH)
(74) Representative: Potter Clarkson
(86) International application number: PCT/EP2010/054748
(87) International publication number: WO 2010/127924

(56) References cited:
- WO-A-97/29733
- WO-A1-00/66072
- WO-A1-00/66072
- WO-A1-00/66080
- WO-A1-2009/138194
- CN-A- 101 112 349
- DE-A1- 10 111 288
- KR-A- 20010 019 410
- US-A1- 2007 110 696

## Description

The present invention relates to a hair treatment composition comprising anti-dandruff agents and a cationic deposition polymer.

DE 101 11 288 (Wella) discloses a composition comprising climbazole, zinc pyrithione and Polyquaternium-10.

Despite the prior art there remains the need for improved anti-dandruff compositions.

Accordingly, the present invention provides a hair treatment composition according to claim 1.

Preferably, the zinc-based antidandruff agent is zinc pyrithione.

The zinc-based antidandruff agent is present at from 0.01 to 1.5 and preferably from 0.75 to 1.25% wt. of the composition.

Preferably, the conazole fungicide is selected from ketoconazole and climbazole.

The conazole fungicide is present at from 0.01 to 2% wt. of the composition. More preferably it is present at from 0.025 to 0.75% wt. of the composition.

Such cationic deposition polymers may be a homopolymer or be formed from two or more types of monomers. The molecular weight of the polymer will generally be between 5 000 and 10 000 000 unified atomic mass units, typically at least 10 000 and preferably from 100 000 to 2 000 000. The polymers will have cationic nitrogen containing groups such as quaternary ammonium or protonated amino groups, or a mixture thereof. A suitable cationic deposition polymer is AQU D4051 a cationically-modified guar commercially available from Aqualon.

Preferably, the cationic deposition polymer is guar hydroxypropyl trimonium chloride.

The compositions according to the invention contain cationic deposition polymer from 0.08 to 0.25% by weight of the composition.

The composition according to the invention may be in any common product form used to treat hair. However, preferably, it is shampoo composition.

The composition according to the invention may comprise any of the ingredients commonly found in hair treatment compositions depending on the product form.

For example, where the composition is a shampoo it will comprise at least one cleansing surfactant such as is commonly employed in shampoos. Where it is a composition which aims to provide conditioning benefit, whether as part of a shampoo composition or a dedicated conditioning composition it will comprise a conditioning active. Suitable conditioning actives include fatty alcohols, silicones and cationic surfactants.

### EXAMPLE 1

The following formulation is an embodiment of the invention and is made by standard processes.

| **Ingredient** | **% wt.** |
|---|---|
| Sodium Laureth Sulphate | 14 |
| Cocoamidopropyl betaine | 1.6 |
| Guar hydroxypropyl trimonium chloride* | 0.2 |
| Zinc Pyrithione | 1.0 |
| Climbazole | 0.5 |
| Zinc sulphate | 0.1 |
| Dimethicone | 2.0 |
| Perfume | 0.7 |
| Sodium chloride | 0.5 |
| Propylene Glocol | 1.0 |
| Acrylic Acid polymer (Carbomer) | 0.6 |
| Polypropylene Glycol | 0.1 |
| Preservative | 0.22 |
| Preservative | 0.09 |
| Water | To 100 |
| * Jaguar C17 | |

### EXAMPLE 2

The following demonstrates the efficacy of climbazole in the presence of cationic guar and polyquaternium-10.

| Test sample | Conc. ZPTO µg/cm3 after one rinse (two rinses) | S. D. |
|---|---|---|
| 1 % ZPTO, 0.5% climbazole, 0.2% Jaguar C17 | 15.51 (12.199) | 1.35 (1.456) |
| 1 % ZPTO, 0.5% climbazole, 0.2% PQ-10 | 12.39 (7.49) | 1.11 (0.57) |

## Claims

1. Hair treatment composition comprising 0.01 to 1.5% of zinc-based antidandruff agent by weight of the composition, 0.01 to 2.00 wt% of a conazole fungicide and from 0.08 to 0.25% of cationic modified guar deposition polymer by weight of the composition, with the proviso that the composition is not a shampoo consisting of:
9 wt.% of sodium lauryl ether sulphate;
2 wt% of cocoamidopropyl betaine;
3.5 wt.% of disodium PEG-5 lauryl citrate sulphosuccinate;
0.5 wt% of polyquaternium-7;
0.2 wt.% of guar hydroxypropyltrimonium chloride;
0.5 wt.% of PEG-40 hydrogenated castor oil,
3 wt.% of glycol distearate;
0.5 wt.% of laureth-9;
0.3 wt.% of panthenol;
3 wt.% of urea;
15 wt.% of PEG-7 glyceryl cocoate;
1 wt.% of dimethicone;
1.25 wt.% Polysaf 5600 (40% strength dispersion);
0.2 wt.% of sodium salicylate;
1 wt.% of zinc pyrithione;
0.5 wt.% of sodium benzoate;
0.5 wt.% of cocamide DEA;
0.5 wt.% of Coolact 10;
0.2 wt.% of Benzophenone-4;
1 wt.% of climbazole,
q.s. of sodium hydroxide solution, perfume, citric acid, and sodium chloride; and
balance of water.

2. Composition according to claim 1 wherein the zinc-based antidandruff agent is zinc pyrithione.

3. Composition according to any preceding claim wherein the conazole fungicide is selected from ketoconazole and climbazole.

## Patentansprüche

1. Haarbehandlungszusammensetzung, die 0,01 bis 1,5 % eines auf Zink basierenden Antischuppenmittels, bezogen auf das Gewicht der Zusammensetzung, 0,01 bis 2,00 Gew.-% eines Conazol-Fungizids und 0,08 bis 0,25 % eines Ablagerungspolymers in Form von kationisch modifiziertem Guar, bezogen auf das Gewicht der Zusammensetzung, umfasst, mit der Maßgabe, dass die Zusammensetzung kein Shampoo ist, das aus Folgendem besteht:
9 Gew.-% Natriumlaurylethersulfat;
2 Gew.-% Cocosamidopropylbetain;
3,5 Gew.-% Dinatrium PEG-5 Laurylcitratsulfosuccinat;
0,5 Gew.-% Polyquaternium-7;
0,2 Gew.-% Guarhydroxypropyl-Trimoniumchlorid;
0,5 Gew.-% hydriertem Rizinusöl-PEG-40;
3 Gew.-% Glycoldistearat;
0,5 Gew.-% Laureth-9;
0,3 Gew.-% Panthenol;
3 Gew.-% Harnstoff;
15 Gew.-% Glycerylcocoat-PEG-7;
1 Gew.-% Dimethicon;
1,25 Gew.-% Polysaf 5600 (40%-ige Dispersion);
0,2 Gew.-% Natriumsalicylat;
1 Gew.-% Zinkpyrithion;
0,5 Gew.-% Natriumbenzoat;
0,5 Gew.-% Cocosamid-DEA;
0,5 Gew.-% Coolact 10;
0,2 Gew.-% Benzophenon-4;
1 Gew.-% Climbazol;
ausreichende Mengen von Natriumhydroxidlösung, Duftstoff, Citronensäure und Natriumchlorid; und
einem Rest in Form von Wasser.

2. Zusammensetzung nach Anspruch 1, wobei das auf Zink basierende Antischuppenmittel Zinkpyrithion ist.

3. Zusammensetzung nach einem vorhergehenden Anspruch, wobei das Conazol-Fungizid aus Ketoconazol und Climbazol ausgewählt ist.

## Revendications

1. Composition de traitement des cheveux comprenant 0,01 à 1,5 % d'agent antipelliculaire à base de zinc en poids de la composition, 0,01 à 2,00 % en poids d'un fongicide conazole et de 0,08 à 0,25 % de polymère de dépôt de guar à modification cationique en poids de la composition, avec la condition que la composition n'est pas un shampoing consistant en :
9 % en poids de lauryléthersulfate de sodium ;
2 % en poids de cocoamidopropylbétaïne ;
3,5 % en poids de PEG-5 laurylcitrate sulfosuccinate de disodium;
0,5 % en poids de polyquaternium-7 ;
0,2 % en poids de chlorure d'hydroxypropyltrimonium de guar ;
0,5 % en poids de PEG-40 huile de ricin hydrogénée,
3 % en poids de distéarate de glycol ;
0,5 % en poids de laureth-9 ;
0,3 % en poids de panthénol ;
3 % en poids d'urée ;
15 % en poids de PEG-7 cocoate de glycéryle ;
1 % en poids de diméthicone ;
1,25 % en poids de Polysaf 5600 (dispersion à 40 %) ;
0,2 % en poids de salicylate de sodium ;
1 % en poids de pyrithione de zinc ;
0,5 % en poids de benzoate de sodium ;
0,5 % en poids de cocamide DEA ;
0,5 % en poids de Coolact 10 ;
0,2 % en poids de benzophénone-4 ;
1 % en poids de climbazole,
q.s. de solution d'hydroxyde de sodium, de parfum, d'acide citrique, et de chlorure de sodium ; et
le complément d'eau.

2. Composition selon la revendication 1 où l'agent antipelliculaire à base de zinc est la pyrithione de zinc.

3. Composition selon l'une quelconque des revendications précédentes où le fongicide conazole est choisi parmi le kétoconazole et le climbazole.
